# EUROPEAN PATENT APPLICATION

(11) **EP 3 892 331 A1**
(43) Date of publication of application: **13.10.2021**
(21) Application number: 19893313.7
(22) Date of filing: 04.12.2019
(51) Int. Cl.: A61P 31/14, A61P 43/00, A61K 35/745, A61K 35/747, A23L 33/135, A23L 33/19, A61K 38/40

(54) **COMPOSITION FOR SUPPRESSING NOROVIRUS INFECTION**

(30) Priority: 07.12.2018 JP 2018230345
(71) Applicant: Morinaga Milk Industry Co., Ltd., Minato-ku Tokyo 108-8384 (JP)
(72) Inventor: ODA, Hirotsugu, Zama-shi, Kanagawa 252-8583 (JP); WAKABAYASHI, Hiroyuki, Zama-shi, Kanagawa 252-8583 (JP); MATSUMOTO, Tetsuya, Tokyo 160-8402 (JP)
(74) Representative: Vossius & Partner Patentanwälte Rechtsanwälte mbB
(86) International application number: PCT/JP2019/047425
(87) International publication number: WO 2020/116511

(57) **Abstract**

An object of this present invention is to provide a composition for inhibiting norovirus infection which can inhibit norovirus from infecting a human, and the object is achieved by a composition for inhibiting norovirus infection comprising one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *Bulgaricus* and *Lactobacillus paracasei* as an active ingredient.

## Description

### Technical Field

This present invention relates to a composition for inhibiting norovirus infection.

### Background Art

Norovirus causes symptoms of acute gastroenteritis such as vomiting and diarrhea in humans and is known to be a virus causing winter gastroenteritis and food poisoning in which the number of cases increases from early fall to early spring. It is known that the main infection route to humans is oral infection and that feces and vomit of infected persons and goods and the like which are directly or indirectly contaminated with the feces and vomit are typical sources of infection.

Research for preventing norovirus infection are carried out in various fields.

For example, it has been reported that an iron-binding glycoprotein which is called lactoferrin and which is contained in exocrine secretions such as breast milk, tears, sweat, and saliva has an effect of preventing infection with norovirus.

Specifically, it has been reported that lactoferrin has an effect of preventing norovirus infection in mice through inhibition of attachment and replication of norovirus (Non-patent document 1).

In addition, it is known that lactoferrin has a protective effect against infection with norovirus in humans (Patent document 1).

Moreover, it has been reported that lactoferrin has an action of inhibiting the onset of gastroenteritis caused by norovirus infection among nursery children (Non-patent document 2).

Furthermore, it is known that specific bacteria of *Bifidobacterium* bacteria and *Lactobacillus* bacteria have certain effects on norovirus.

For example, it has been reported that a bacterium belonging to *Bifidobacterium adolescentis,* which is one of *Bifidobacterium* bacteria, inhibits the growth of murine norovirus (Non-patent document 3).

Moreover, it has been reported that continuous intake of a drink containing *Lactobacillus casei* strain Shirota, which is a species of *Lactobacillus* bacteria, relieves fever after the onset of gastroenteritis caused by norovirus infection (Non-patent document 4).

However, it has not been previously reported that *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *Bulgaricus,* and/or *Lactobacillus paracasei* can inhibit infection of norovirus in humans.

### Citation List

### Patent Literature

Patent Document 1: JP 2018-111658A

### Non Patent Literature

Non-patent document 1: Biochem. Biophys. Res. Commun., 434 (2013) 791-796
Non-patent document 2: Japanese Journal of Complementary and Alternative Medicine, Vol. 9, No. 2, September, 2012: 121-128
Non-patent document 3: Front Microbiol., 2016; 7: 864
Non-patent document 4: Br. J. Nutr., 106, p549-556 (2011)

### Summary of Invention

### Technical Problem

An object of the present invention is to provide a composition for inhibiting norovirus infection which can inhibit norovirus from infecting a human.

### Solution to Problem

The inventors have found that specific bacteria of *Bifidobacterium* bacteria and specific bacteria of *Lactobacillus* bacteria can inhibit infection of norovirus in a human and have thus completed the invention.

That is, the present invention provides a composition for inhibiting norovirus infection which comprises one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *Bulgaricus* and *Lactobacillus paracasei* as an active ingredient.

In a preferable embodiment of the composition, *Bifidobacterium breve* is *Bifidobacterium breve* NITE BP-02622 or *Bifidobacterium breve* FERM BP-11175.

In a preferable embodiment of the composition, *Bifidobacterium longum* subsp. *infantis* is *Bifidobacterium longum* subsp. *infantis* NITE BP-02623.

In a preferable embodiment of the composition, *Lactobacillus paracasei* is *Lactobacillus paracasei* NITE BP-01633.

In a preferable embodiment, the composition comprises lactoferrin.

In a preferable embodiment, the composition is a pharmaceutical composition.

In a preferable embodiment, the composition is a food or drink composition.

### Advantageous Effects of Invention

The composition for inhibiting norovirus infection of the present invention can inhibit norovirus from infecting a human.

### Brief Description of Drawings

Fig. 1 shows a graph showing experimental results of inhibition of MNV infection by *Bifidobacterium breve* M-16V (NITE BP-02622) in the Examples of the present invention.
Fig. 2 shows a graph showing experimental results of inhibition of MNV infection by *Bifidobacterium breve* MCC1274 (FERM BP-11175) in the Examples of the present invention.
Fig.3 shows a graph showing experimental results of inhibition of MNV infection by *Bifidobacterium longum* subsp. *infantis* M-63 (NITE BP-02623) in the Examples of the present invention.
Fig. 4 shows a graph showing experimental results of inhibition of MNV infection by *Lactobacillus paracasei* MCC1849 (NITE BP-01633) in the Examples of the present invention.

### Description of Embodiments

The present invention is explained in detail below.

The composition for inhibiting norovirus infection of the present invention comprises one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *Bulgaricus* and *Lactobacillus paracasei* as an active ingredient.

*Bifidobacterium longum* subsp. *infantis* is sometimes simply referred to as *Bifidobacterium infantis.*

*Lactobacillus delbrueckii* subsp. *bulgaricus* is sometimes simply referred to as *Lactobacillus bulgaricus.*

The bacteria that the composition for inhibiting norovirus infection of the present invention comprises as an active ingredient are sometimes referred to as "the bacteria (or bacterium) of the present invention" below.

Moreover, the composition is sometimes referred to as "the composition of the present invention" below. In this regard, the composition of the present invention is a concept including a mixture, and the components may be homogeneous or heterogeneous.

The bacteria of the present invention, which are the active ingredient of the composition of the present invention, have an action of inhibiting a human who takes the bacteria from being infected with norovirus. In this specification, "inhibiting a human from being infected with norovirus" is sometimes referred to as "inhibiting norovirus infection".

*Bifidobacterium breve* of the present invention is preferably *Bifidobacterium breve* NITE BP-02622, *Bifidobacterium breve* FERM BP-11175 or *Bifidobacterium breve* ATCC 15700.

*Bifidobacterium longum* subsp. *infantis* of the present invention is preferably *Bifidobacterium longum* subsp. *infantis* NITE BP-02623 or *Bifidobacterium longum* subsp. *infantis* BCCM LMG23728.

*Lactobacillus helveticus* of the present invention is preferably *Lactobacillus helveticus* NITE BP-01671.

*Bifidobacterium bifidum* of the present invention is preferably *Bifidobacterium bifidum* NITE BP-02429, *Bifidobacterium bifidum* NITE BP-02431, *Bifidobacterium bifidum* NITE BP-02432 or *Bifidobacterium bifidum* NITE BP-02433.

*Lactobacillus paracasei* of the present invention is preferably *Lactobacillus paracasei* NITE BP-01633.

The bacterium to which the accession number of NITE BP-02622 was given was deposited as an international deposit under the Budapest Treaty on January 26, 2018 to the NITE Patent Microorganisms Depositary, National Institute of Technology and Evaluation (Room 122, 2-5-8 Kazusakamatari, Kisarazu-shi, Chiba 292-0818) and given the accession number of NITE BP-02622. The bacterium is the same bacterium as *Bifidobacterium breve* M-16V.

The bacterium to which the accession number of FERM BP-11175 was given was deposited as an international deposit under the Budapest Treaty on August 25, 2009 to International Patent Organism Depositary, National Institute of Advanced Industrial Science and Technology (current International Patent Organism Depositary, National Institute of Technology and Evaluation, Room 120, 2-5-8 Kazusakamatari, Kisarazu-shi, Chiba 292-0818). The bacterium is the same bacterium as *Bifidobacterium breve* MCC1274.

*Bifidobacterium breve* ATCC 15700 can be acquired from the American Type Culture Collection (address: 12301 Parklawn Drive, Rockville, Maryland 20852, United States of America).

The bacterium to which the accession number of NITE BP-02623 was given was deposited as an international deposit under the Budapest Treaty on January 26, 2018 to NITE Patent Microorganisms Depositary, National Institute of Technology and Evaluation (Room 122, 2-5-8 Kazusakamatari, Kisarazu-shi, Chiba 292-0818) and given the accession number of NITE BP-02623. The bacterium is the same bacterium as *Bifidobacterium longum* subsp. *infantis* M-63.

*Bifidobacterium longum* subsp. *infantis* BCCM LMG23728 can be acquired from Belgian Coordinated Collections of Microorganisms (BCCM) (address: Rue de la Science (Wetenschapsstraat) 8, Brussels B-1000, Belgium), which is a depositary authority in Belgium.

The bacterium to which the accession number of NITE BP-01671 was given was deposited as an international deposit under the Budapest Treaty on July 29, 2013 to NITE Patent Microorganisms Depositary, National Institute of Technology and Evaluation (Room 122, 2-5-8 Kazusakamatari, Kisarazu-shi, Chiba 292-0818) and given the accession number of NITE BP-01671. The bacterium is the same bacterium as *Lactobacillus helveticus* MCC1848.

The bacterium to which the accession number of NITE BP-02429 was given was deposited as an international deposit under the Budapest Treaty on February 21, 2017 to NITE Patent Microorganisms Depositary, National Institute of Technology and Evaluation (Room 122, 2-5-8 Kazusakamatari, Kisarazu-shi, Chiba 292-0818) and given the accession number of NITE BP-02429. The bacterium is the same bacterium as *Bifidobacterium bifidum* MCC1092.

The bacterium to which the accession number of NITE BP-02431 was given was deposited as an international deposit under the Budapest Treaty on February 21, 2017 to NITE Patent Microorganisms Depositary, National Institute of Technology and Evaluation (Room 122, 2-5-8 Kazusakamatari, Kisarazu-shi, Chiba 292-0818) and given the accession number of NITE BP-02431. The bacterium is the same bacterium as *Bifidobacterium bifidum* MCC1319.

The bacterium to which the accession number of NITE BP-02432 was given was deposited as an international deposit under the Budapest Treaty on February 21, 2017 to NITE Patent Microorganisms Depositary, National Institute of Technology and Evaluation (Room 122, 2-5-8 Kazusakamatari, Kisarazu-shi, Chiba 292-0818) and given the accession number of NITE BP-02432. The bacterium is the same bacterium as *Bifidobacterium bifidum* MCC1868.

The bacterium to which the accession number of NITE BP-02433 was given was deposited as an international deposit under the Budapest Treaty on February 21, 2017 to NITE Patent Microorganisms Depositary, National Institute of Technology and Evaluation (Room 122, 2-5-8 Kazusakamatari, Kisarazu-shi, Chiba 292-0818) and given the accession number of NITE BP-02433. The bacterium is the same bacterium as *Bifidobacterium bifidum* MCC1870.

The bacterium to which the accession number of NITE BP-01633 was given was deposited on June 6, 2013 to NITE Patent Microorganisms Depositary, National Institute of Technology and Evaluation (Room 122, 2-5-8 Kazusakamatari, Kisarazu-shi, Chiba 292-0818), given the accession number of NITE P-01633, converted to an International Depositary Authority under the Budapest Treaty on December 19, 2013, and given the accession number of NITE BP-01633. The bacterium is the same bacterium as *Lactobacillus paracasei* MCC1849.

*Bifidobacterium breve* NITE BP-02622 is not limited to the deposited bacterium and may be a substantially equivalent bacterium to the deposited bacterium. A bacterium which is substantially equivalent to the deposited bacterium is as follows: the bacterium is a bacterium belonging to *Bifidobacterium breve;* a human can exert an action of inhibiting norovirus infection when the human takes the bacterium; the nucleotide sequence of 16SrRNA gene thereof has a homology of preferably 98% or higher, more preferably 99% or higher, further preferably 100% to the nucleotide sequence of 16SrRNA gene of the deposited bacterium; and the bacterium is preferably a bacterium having the same mycological properties as those of the deposited bacterium. The bacterium also includes a mutant and a gene recombinant strain derived from the bacterium.

The same applies to the other deposited bacteria above.

The active ingredient of the composition of the present invention may be bacterial cells of any of the bacteria above. A culture obtained after cultivation may be used directly or used after dilution or concentration, or bacterial cells collected from a culture may also be used. Moreover, as long as the effects of the present invention are not impaired, the bacteria of the present invention can be subjected to various additional operations such as heating and freeze-drying after cultivation. The bacteria of the present invention may be living cells or dead cells or may be both living cells and dead cells. The dead cells are dead cells which have been sterilized by heating or the like or may be an homogenate of bacteria.

The culture of a bacterium is a medium used for culturing the bacterium, a concentrate or a dried material of the medium, or what is obtained by fractionating or purifying a fraction or a component which can inhibit a human from being infected with norovirus from the medium.

A bacterium of the present invention can be easily grown by culturing the bacterium. The method for cultivation is not particularly limited as long as the bacterium can grow, and a method which is generally used for culturing *Bifidobacterium* bacteria (bifidobacteria) and *Lactobacillus* bacteria (lactic acid bacteria) can be used with appropriate modification when necessary. For example, the culture temperature may be 25 to 50°C and is preferably 30 to 40°C. Moreover, cultivation is conducted preferably under anaerobic conditions, and for example, cultivation can be conducted while flowing an anaerobic gas such as carbon dioxide. Furthermore, cultivation may be conducted under microaerophilic conditions such as a static liquid culture.

The medium used for cultivation is not particularly limited, and a medium which is generally used for culturing a *Bifidobacterium* bacterium or a *Lactobacillus* bacterium can be used with appropriate modification when necessary. That is, as a carbon source, for example, saccharides such as galactose, glucose, fructose, mannose, cellobiose, maltose, lactose, sucrose, trehalose, starch, starch hydrolysate and molasses can be used depending on the assimilation properties. As a nitrogen source, for example, ammonia and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, ferrous sulfate and the like can be used. Furthermore, organic components such as peptone, soybean powder, a defatted soybean cake, meat extract and yeast extract may also be used. In addition, as a modified medium, for example, MRS medium can be preferably used.

The composition of the present invention preferably comprises lactoferrin. Lactoferrin may be lactoferrin derived from the milk of a mammal, lactoferrin isolated by a general method from defatted milk, whey and the like, which are processed milk, recombinant lactoferrin produced from a microorganism, animal cells, a transgenic animal or the like by gene manipulation, synthetic lactoferrin, or a mixture thereof. Moreover, lactoferrin may be non-glycosylated or glycosylated.

Examples of lactoferrin used in the present invention are metal-saturated lactoferrin, partially metal-saturated lactoferrin and apolactoferrin, and one or more of the lactoferrin types can be used in the present invention. Moreover, the lactoferrin content of the composition of the present invention is not particularly limited as long as the infection of a human who takes the composition of the present invention with norovirus can be inhibited, but the total amount is preferably in the range of 10 µg/ml to 1 mg/ml, more preferably in the range of 50 µg/ml to 500 µg/ml and further preferably in the range of 100 µg/ml to 300 µg/ml.

The composition of the present invention can be used widely as a pharmaceutical composition and a food or drink composition. For example, a pharmaceutical composition for inhibiting norovirus infection and a food or drink composition for inhibiting norovirus infection can be provided. The compositions are sometimes referred to as "the pharmaceutical composition of the present invention" and "the food or drink composition of the present invention", respectively, below.

The pharmaceutical composition of the present invention is not particularly limited as long as a bacterium of the present invention is present. As the pharmaceutical composition of the present invention, a bacterium of the present invention may be used directly or used after blending with a physiologically acceptable liquid or solid carrier for formulation and forming into a drug.

The dosage form of the pharmaceutical composition of the present invention is not particularly limited, and specifically, examples are tablets, pills, powder, liquid preparation, suspensions, emulsions, granules, capsules, syrups, suppositories, injections, ointment, patches, eye drops, nasal drops and the like. Moreover, for the formulation, an additive which is generally used as a carrier for formulation, such as excipients, binders, disintegrating agents, lubricants, stabilizers, flavoring agents, diluents, surfactants or solvents for injection, can be used.

As the carrier for formulation, various organic or inorganic carriers can be used depending on the dosage form. Examples of the carrier in the case of solid preparation include excipients, binders, disintegrating agents, lubricants, stabilizers, flavoring agents, and the like.

Examples of the excipients include saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of the binders include, in addition to the excipients gelatin; polyvinylpyrrolidone; macrogol; and the like.

Examples of the disintegrating agents include, in addition to the excipients, chemically modified starch or cellulose derivatives such as croscarmellose sodium, sodium carboxymethyl starch and cross-linked polyvinylpyrrolidone and the like.

Examples of the lubricants include talc; stearic acid; metal stearates such as calcium stearate and magnesium stearate; colloidal silica; Veegum; waxes such as spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of the stabilizers include paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of the flavoring agents include sweeteners, acidulants, aromas and the like.

In this regard, the carriers used in the case of a liquid preparation for oral administration include solvents such as water, flavoring agents, and the like.

The amount of the bacterium (bacteria) of the present invention comprised in the pharmaceutical composition of the present invention is appropriately set based on the dosage form, the usage, the age of the subject, the gender, the type of syndrome or symptom, the degree thereof, the other conditions and the like, but in general, the amount is preferably in the range of 1×10⁴ to 1×10¹³ cfu/g or 1×10⁴ to 1×10¹³ cfu/ml, more preferably in the range of 1×10⁵ to 1×10¹² cfu/g or 1×10⁵ to 1×10¹² cfu/ml, further preferably in the range of 1×10⁶ to 1×10¹¹ cfu/g or 1×10⁶ to 1×10¹¹ cfu/ml. The unit "cfu" indicates the colony forming unit. When the bacterium (bacteria) of the present invention is dead cells, cfu/g or cfu/ml can be replaced with cells/g or cells/ml.

The dosage of the pharmaceutical composition of the present invention administered to a subject is appropriately set based on the dosage form, the usage, the subject, the age of the subject, the gender, the type of disease or the like, the degree thereof, other conditions and the like, but the dosage is not particularly limited as long as an action of inhibiting norovirus infection is exerted in the subject to which the pharmaceutical composition is administered. The amount of the bacterium (bacteria) of the present invention per day per kg body weight is preferably in the range of 1×10⁴ to 1×10¹³ cfu, more preferably in the range of 1×10⁵ to 1×10¹² cfu, further preferably in the range of 1×10⁶ to 1×10¹² cfu. When the bacterium (bacteria) of the present invention is dead cells, cfu can be replaced with the individual cells.

The timing of administration of the pharmaceutical composition of the present invention is not particularly limited, and the timing of administration can be appropriately chosen according to the method for preventing or the method for treating the target syndrome or symptom. In this regard, in this specification, "treatment" includes "relief".

The pharmaceutical composition of the present invention may be administered prophylactically before norovirus infection, administered for inhibiting further norovirus infection after norovirus infection, or used as a maintenance therapy. Moreover, the form of administration is preferably determined based on the formulation form, the age of the subject, the gender, the other conditions, the degree of the syndrome or the symptom of the subject or the like. In this regard, in all the cases, the pharmaceutical composition of the present invention can be administered once a day or in separate portions, or may be administered once in several days or in several weeks.

The pharmaceutical composition of the present invention may be administered alone or may be used in combination with another pharmaceutical composition, a medicine, a food or drink composition or a food or a drink: such as, for example, another pharmaceutical composition, a medicine, a food or drink composition or a food or a drink for inhibiting norovirus infection; a pharmaceutical composition, a medicine, a food or drink composition or a food or a drink for a syndrome or a symptom which can be prevented or treated by inhibiting norovirus infection; or the like. Examples of the syndrome or the symptom include nausea, vomiting, diarrhea, abdominal pain, fever, and the like.

The food or drink composition of the present invention is not particularly limited as long as the composition comprises a bacterium of the present invention. The food or drink composition of the present invention is not limited regarding the form such as liquid, paste, gel solid, or powder and may be a food or a drink, a tablet candy, a liquid food, or the like as well as the following examples: wheat products such as breads, macaroni, spaghetti, noodles, cake mixes, frying flours, and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods, and other instant foods; processed agricultural products such as canned agricultural products, canned fruits, jams/marmalades, pickles, cooked beans, dried agricultural products, and cereals (processed grains); processed fishery products such as canned fishery products, fish hams/sausages, fishery paste products, fishery delicacies, and *Tsukudani* (foods boiled down in sweetened soy sauce); processed livestock products such as canned livestock products/pastes and livestock hams/sausages; milk/dairy products such as processed milk, milk beverages, yogurts, lactic acid bacteria beverages, cheeses, ice creams, modified milk powders, creams, and other dairy products; oils and fats such as butter, margarine, and vegetable oils; basic condiments such as soy sauce, soybean paste, sauces, processed tomato condiments, *Mirin* (sweet sake for seasoning), and vinegars; compound flavor enhancers/foods such as cooking mixes, curry roux, sauces, dressings, noodle broths, spices, and other compound flavor enhancers; frozen foods such as frozen food materials, semi-cooked frozen foods and cooked frozen foods; confectioneries such as caramels, candies, gummy candies, chewing gums, chocolates, cookies, biscuits, cakes, pies, snacks, crackers, Japanese-style confectioneries, rice confectioneries, bean confectioneries, desserts, jellies, and other confectioneries; luxury beverages such as carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols, and other luxury beverages, other commercial foods such as baby foods, *Furikake* (dry Japanese seasonings), and seasonings for *Chazuke* (boiled rice with hot tea); infant modified milk powder; enteral nutrition products; food for special dietary uses and food with health claims (foods for specified health uses, foods with nutrient function claims, and foods with function claims); nutritional supplements; and the like.

Moreover, the food or drink composition of the present invention may be a supplement and may be, for example, a supplement in tablet form. In the case of a supplement, the bacterium (bacteria) of the present invention can be taken while the amount of meals and the calorie intake per day are not affected by other foods.

The food or drink composition of the present invention can be produced by adding a bacterium of the present invention to a general material of a food or a drink and can be produced in the same manner as that of a general food or drink except that a bacterium of the present invention is added. A bacterium of the present invention may be added in any stage of the production process of the food or drink composition. Moreover, the food or drink composition of the present invention may be produced through a fermentation process by the added bacterium of the present invention. Such food or drink compositions are lactic acid bacteria beverages, fermented milk, and the like.

As the material of the food or drink composition of the present invention, materials which are used for general foods and drinks can be used. The produced food or drink composition can be orally taken.

The food or drink composition of the present invention also includes a material which is added to a food or drink composition during the production process of a food or drink composition or after the production, such as a material for producing a food or drink composition, a food additive, and the like. For example, a bacterium of the present invention can be used as a starter for producing fermented milk. Moreover, a bacterium of the present invention can be added later to produced fermented milk.

In the food or drink composition of the present invention, a component which has a prebiotic effect which is known or will be found in the future, or a component which supplements a prebiotic effect can be used as long as the effects of the present invention are not impaired. For example, the food or drink composition of the present invention can be produced by blending a bacterium of the present invention with a component such as various proteins such as whey protein, casein protein, soybean protein, pea protein, or mixtures or decomposition products thereof; amino acids such as leucine, valine, isoleucine, or glutamine; vitamins such as vitamin B6 or vitamin C; creatine; citric acid; fish oil; or oligosaccharides such as isomaltooligosaccharides, galactooligosaccharides, xylooligosaccharides, soybean oligosaccharides, fructooligosaccharides, lactulose, and HMOs (human milk oligosaccharides).

Human milk oligosaccharides are 2'-fucosyllactose, 3-fucosyllactose, 2',3-difucosyllactose, 3'-sialyllactose, 6'-sialyllactose, 3-fucosyl-3'-sialyllactose, lacto-N-tetraose, lacto-N-neotetraose, lacto-N-fucopentaose I, lacto-N-fucopentaose II, lacto-N-fucopentaose III, lacto-N-fucopentaose V, lacto-N-difucosylhexaose I, lacto-N-difucosylhexaose II, lacto-N-sialylpentaose, LSTa, LSTb, LSTc, and the like.

The amount of the bacterium (bacteria) of the present invention present in the food or drink composition of the present invention is appropriately set based on the form of the food or drink composition, but in general, the amount in the food or drink composition is preferably in the range of 1×10⁴ to 1×10¹³ cfu/g or 1×10⁴ to 1×10¹³ cfu/ml, more preferably in the range of 1×10⁵ to 1×10¹² cfu/g or 1×10⁵ to 1×10¹² cfu/ml, further preferably in the range of 1×10⁶ to 1×10¹¹ cfu/g or 1×10⁶ to 1×10¹¹ cfu/ml. The unit "cfu" indicates the colony forming unit. When the bacterium (bacteria) of the present invention is dead cells, cfu/g or cfu/ml can be replaced with cells/g or cells/ml.

The intake of the food or drink composition of the present invention is appropriately set based on the form of the food or drink composition, the usage, the subject, the age of the subject, the gender, other conditions and the like but is not particularly limited as long as an action of inhibiting norovirus infection is exerted in the subject who takes the food or drink composition. The amount of the bacterium (bacteria) of the present invention per day per kg body weight is preferably in the range of 1×10⁴ to 1×10¹³ cfu, more preferably in the range of 1×10⁵ to 1×10¹² cfu, further preferably in the range of 1×10⁶ to 1×10¹² cfu. When the bacterium (bacteria) of the present invention is dead cells, cfu can be replaced with the individual cells.

In this regard, the food or drink composition of the present invention can be taken once a day or in separate portions. The food or drink composition may be taken once in several days or in several weeks but is preferably taken every day.

The food or drink composition of the present invention may be taken alone or taken together with another food or drink composition, a food or a drink, a pharmaceutical composition or a medicine such as, for example, another food or drink composition, a food or a drink, a pharmaceutical composition or a medicine for inhibiting norovirus infection; a food or drink composition, a food or a drink, a pharmaceutical composition or a medicine for a syndrome or a symptom which can be prevented or treated by inhibiting norovirus infection; or the like. Examples of the syndrome or the symptom include nausea, vomiting, diarrhea, abdominal pain, fever, and the like.

The food or drink composition of the present invention can be sold as a food or drink composition or a food or a drink with a label of use for inhibiting norovirus infection. Moreover, the food or drink composition of the present invention can be sold as a food or drink composition or a food or a drink with a label of use for preventing or treating a syndrome or a symptom which can be prevented or treated by inhibiting norovirus infection. Furthermore, the food or drink composition or the food or the drink of the present invention can be labeled with "for inhibiting norovirus infection" or the like. In addition, of course, a term can be used as long as the term indicates a secondary effect caused by inhibiting norovirus infection.

The food or drink composition of the present invention can be provided/sold as a food or drink composition or a food or a drink with a label of use as probiotics and the like (including health uses) . Moreover, the food or drink composition or the food or the drink can be provided/sold with a label for "those who wish to lead a life with bifidobacteria", "those who wish to lead a life with lactic acid bacteria", "those who want to improve the intestinal environment", "those who want to correct the stomach condition", "those who want to form a good intestinal environment" or the like as the subject of the consumption.

The "label" means all the acts for informing a consumer of the use, and all the labels which remind of/cause to guess the uses are the "labels" of the present invention, regardless of the purposes of the labels, the contents of the labels, the objects to be labeled, the media and the like. However, labeling with an expression which allows a consumer to directly recognize the use is preferable.

Specifically, examples are an act of describing the use on a product regarding the food or drink composition or the food or the drink of the present invention or on packaging of a product, an act of transferring an article in which the use is described on a product or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and other acts, and in particular, labeling on packaging, a container, a catalogue, a brochure, an advertisement material in a sales site such as POP, other documents or the like is preferable.

The label is preferably a label approved by the administration or the like (for example, a label approved based on a system provided by the administration and provided in the form based on the approval). Examples include labels with food with health claims, more specifically food with health claims, health foods, functional foods, enteral nutrition products, food for special dietary uses, foods with nutrient function claims, quasi-drugs or the like, and other examples include labels approved by the Consumer Affairs Agency, such as foods for specified health uses, foods with nutrient function claims, foods with function claims, and labels approved by a similar system. Examples of the latter include a label with foods for specified health uses, a label with qualified foods for specified health uses, a label indicating influence on the structure or the function of a body, a label with reduction of disease risk, a label with a scientifically grounded function and the like. More specifically, examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009), similar labels and the like.

An example of the food or drink composition of the present invention is infant milk (for example, infant modified milk powder and the like) . The "infant milk" means a food which is designed in a way that an infant, preferably 0 to 36 months of age, more preferably 0 to 12 months of age, can drink as a breast milk substitute and which alone meets the nutritional demand of an infant. The infant milk can comprise a bacterium of the present invention and/or lactoferrin; a prebiotic such as human milk oligosaccharides, fructooligosaccharides, and galactooligosaccharides; protein derived from casein, soybeans, whey, or skimmed milk; a carbohydrate such as lactose, saccharose, maltodextrins, starch, or a mixture thereof; fat (for example, palmolein, sunflower oil, or sunflower oil); a vitamin and a mineral which are essential in daily foods; and the like, and the infant milk can comprise one, two or more kinds selected from the group.

The present invention can also employ the following structures.
[1] Use of one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* in the manufacture of a composition for inhibiting norovirus infection.
[2] Use of one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for the inhibition of norovirus infection.
[3] One or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for use in the inhibition of norovirus infection.
[4] One or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for use in the prevention or treatment of a syndrome or a symptom which can be prevented or treated by inhibiting norovirus infection.
[5] A method for inhibiting norovirus from infecting a human, including a step of administering one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* or a composition for inhibiting norovirus infection to a human.
[6] A method for preventing or a method for treating a syndrome or a symptom which can be prevented or treated by inhibiting norovirus infection, including a step of administering one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* or a composition for inhibiting norovirus infection to a human.

### Examples

Although the present invention is explained specifically below using Examples, the present invention is not limited to the Examples.

### [Preparation Example 1]

### (Preparation of Bacteria)

Dead cells of the following four kinds of bacterium were prepared:
- *Bifidobacterium breve* M-16V (NITE BP-02622)
- *Bifidobacterium breve* MCC1274 (FERM BP-11175)
- *Bifidobacterium longum* subsp. *infantis* M-63 (NITE BP-02623)
- *Lactobacillus paracasei* MCC1849 (NITE BP-01633)

Specifically, dead cells of the *Bifidobacterium* bacteria were prepared by anaerobically culturing in MRS medium (containing 0.05% cysteine), heat treating at 70°C for 10 minutes, then removing the supernatant and washing with PBS. The culture solutions before and after the heat treatment were smeared on TOS propionate agar medium, and the number of cells were checked.

Dead cells of the *Lactobacillus* bacterium were prepared by culturing in MRS medium (containing 0.5% lactose), heat treating at 70°C for 10 minutes, then removing the supernatant and washing with PBS. The culture solutions before and after the heat treatment were smeared on BCP-added agar medium plates, and the numbers of the cells were checked.

### (Preparation of Virus)

Murine norovirus-1 CW1 (sometimes referred to as MNV below) provided by Professor H. W. Virgin (Washington University School of Medicine, the U.S.) was proliferated according to a general method using macrophage-like RAW264.7 cells (ATCC TIB-71, Summit Pharmaceuticals International Corporation), and the virus at a concentration of 1×10⁷ pfu/ml was obtained. When the virus was used, the virus was diluted and used.

### (Preparation of Cells)

RAW264.7 cells (ATCC TIB-71, Summit Pharmaceuticals International Corporation) were cultured using DMEM high glucose medium (Sigma-Aldrich) to which FBS was added at 10% at 37°C in 5% CO₂ with a standard passage number of approximately 15, and the cells were maintained and used.

### (Preparation of Lactoferrin)

Lactoferrin (sometimes abbreviated to LF in this specification) was dissolved in a phosphate buffer to 10 mg/ml and prepared through Millex filter of 0.45 µm. When lactoferrin was used, lactoferrin was diluted to a concentration of 200 µg/ml using 10% FBS DMEM medium and used.

### [Test Example 1] Examination of Effects of Bacteria of Inhibiting MNV Infection

### (Test Method)

Test solutions (1) to (4) below were prepared for each of the four kinds of bacterium above, and the degrees of inhibition of MNV infection of RAW264.7 cells were tested using a plaque assay.
(1) 10% FBS DMEM medium
(2) 10% FBS DMEM medium containing dead cells at 1×10⁵ cells/ml
(3) 10% FBS DMEM medium containing 200 µg/ml lactoferrin
(4) 10% FBS DMEM medium containing dead cells at 1×10⁵ cells/ml and 200 µg/ml lactoferrin

The supernatants of RAW264.7 cells which were cultured for a day were removed, and the cells were precultured with the test solutions from 30 minutes before the infection. After 30 minutes, the test solutions were removed, and the cells were rinsed with DMEM medium. MNV-containing solutions which were diluted with the test solutions were added, and the cells were infected for an hour. Then, the infection solutions were removed, and 0.35% agarose solutions prepared with the test solutions were added. The cells were cultured in an incubator at 37°C with 5% CO₂ for two days and stained, and the plaque numbers were counted.

### (Results)

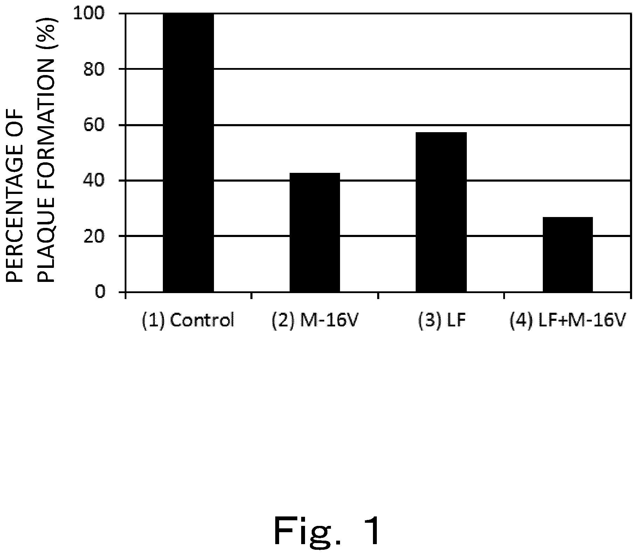

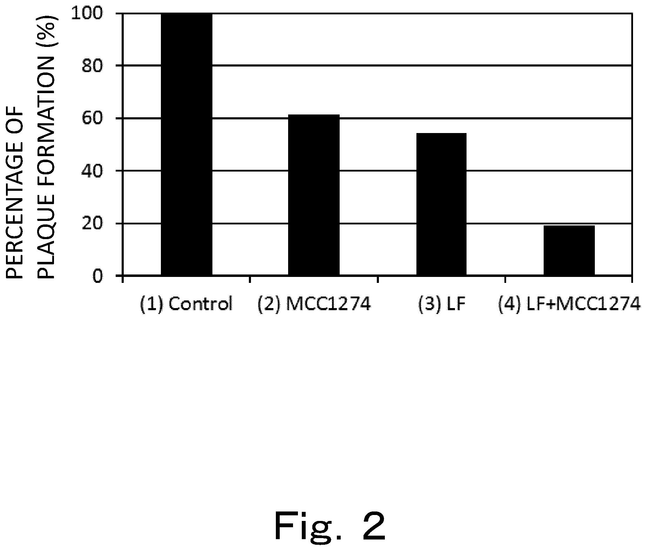

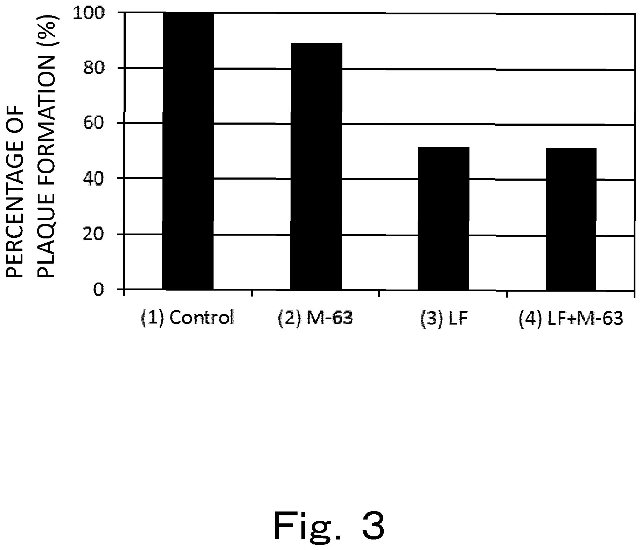

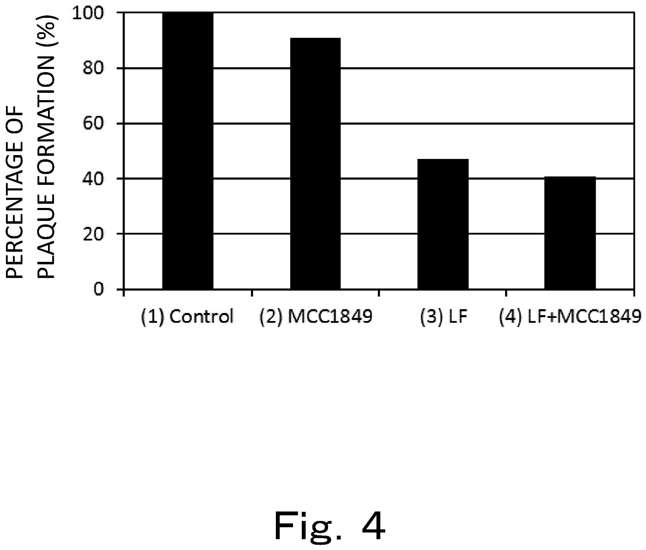

The percentages of plaque formation of the bacteria using the test solutions, where the plaque formation rates of the cases using test solution (1) are regarded as 100%, are shown in Fig. 1 to Fig. 4.

In all of the experiments using the bacteria, as compared to when using the test solution (1) (10% FBS DMEM medium), an effect of inhibiting MNV infection was observed when test solution (2) (10% FBS DMEM medium containing dead cells at 1×10⁵ cells/ml) was used. Moreover, when test solution (4) (10% FBS DMEM medium containing dead cells at 1×10⁵ cells/ml and 200 µg/ml lactoferrin) was used, a stronger or equivalent effect of inhibiting MNV infection was observed as compared with when using test solution (3) (10% FBS DMEM medium containing 200 µg/ml lactoferrin). That is, it was confirmed that the four kinds of bacterium above have an effect of inhibiting MNV infection and that the effect is stronger when lactoferrin is used in combination.

### [Production Example 1]

*Bifidobacterium breve* M-16V (NITE BP-02622), *Bifidobacterium breve* MCC1274 (FERM BP-11175), *Bifidobacterium longum* subsp. *infantis* M-63 (NITE BP-02623), or *Lactobacillus paracasei* MCC1849 (NITE BP-01633) is added to 3 mL of MRS liquid medium and anaerobically cultured at 37°C for 16 hours, and then the bacterial cells are washed three times by centrifugation and re-suspension in distilled water, then suspended in distilled water to 10 mg (in terms of dry cell weight) /ml and sterilized by heating at 100°C for 15 minutes. A solution containing heat-sterilized cells is thus obtained. The solution containing the heat-sterilized cells is concentrated and freeze-dried, and a freeze-dried powder of the bacterium (bacterial powder) is thus obtained. The bacterial powder is mixed evenly with whey protein concentrate (WPC), and a composition is thus obtained. The composition in an amount of 20 g is dissolved in 200 g of water, and a composition for inhibiting norovirus infection is thus obtained.

An effect of inhibiting norovirus infection can be expected through the administration of this composition. Moreover, the composition can be used for preventing or treating a syndrome or a symptom which can be prevented or treated by inhibiting norovirus infection. Examples of the syndrome or the symptom include nausea, vomiting, diarrhea, abdominal pain, fever, and the like.

### [Production Example 2]

*Bifidobacterium breve* M-16V (NITE BP-02622), *Bifidobacterium breve* MCC1274 (FERM BP-11175), *Bifidobacterium longum* subsp. *infantis* M-63 (NITE BP-02623), or *Lactobacillus paracasei* MCC1849 (NITE BP-01633) is added to 3 mL of MRS liquid medium and anaerobically cultured at 37°C for 16 hours, and then the bacterial cells are washed three times by centrifugation and re-suspension in distilled water, then suspended in distilled water to 10 mg (in terms of dry cell weight) /ml and sterilized by heating at 100°C for 15 minutes. A solution containing heat sterilized cells is thus obtained. The solution containing heat sterilized cells is concentrated and freeze-dried, and freeze-dried powder of the bacterium (bacterial powder) is thus obtained. Next, crystalline cellulose is introduced into an agitation granulating machine and mixed. Then, purified water is added, and granules are formed and dried. Granules which contain extract components of the bacterium and which contain an excipient are obtained.

An effect of inhibiting norovirus infection can be expected through the administration of this composition. Moreover, the composition can be used for preventing or treating a syndrome or a symptom which can be prevented or treated by inhibiting norovirus infection. Examples of the syndrome or the symptom include nausea, vomiting, diarrhea, abdominal pain, fever, and the like.

### [Production Example 3]

*Bifidobacterium breve* M-16V (NITE BP-02622), *Bifidobacterium breve* MCC1274 (FERM BP-11175), *Bifidobacterium longum* subsp. *infantis* M-63 (NITE BP-02623) or *Lactobacillus paracasei* MCC1849 (NITE BP-01633) is added to 3 mL of MRS liquid medium and anaerobically cultured at 37°C for 16 hours, and then the bacterial cells are washed three times by centrifugation and re-suspension in distilled water, then suspended in distilled water to 10 mg (in terms of dry cell weight) /ml and sterilized by heating at 100°C for 15 minutes. A solution containing heat sterilized cells is thus obtained. The solution containing heat sterilized cells is concentrated and freeze-dried, and a freeze-dried powder of the bacterium (bacterial powder) is thus obtained. The bacterial powder and an oligosaccharide are mixed evenly, and a composition is thus obtained. The composition is provided as a prebiotic material. The intake of the bacterium is adjusted to 1×10⁴ to 1×10¹³ cfu/kg body weight/day, and the composition is provided at breakfast every day for a week. When the bacterium is dead cells, cfu/kg body weight/day can be replaced with cells/kg body weight/day. In this regard, the composition may be mixed with a food or a drink such as fermented milk. As the oligosaccharide, isomaltooligosaccharides, lactulose, raffinose, fructooligosaccharides, galactooligosaccharides, and soybean oligosaccharide can be used.

An effect of inhibiting norovirus infection can be expected through the administration of this composition. Moreover, the composition can be used for preventing or treating a syndrome or a symptom which can be prevented or treated by inhibiting norovirus infection. Examples of the syndrome or the symptom include nausea, vomiting, diarrhea, abdominal pain, fever, and the like.

### [Production Example 4]

A method for producing fermented milk to which *Bifidobacterium breve* M-16V (NITE BP-02622), *Bifidobacterium breve* MCC1274 (FERM BP-11175), *Bifidobacterium longum* subsp. *infantis* M-63 (NITE BP-02623), or *Lactobacillus paracasei* MCC1849 (NITE BP-01633) is added is shown below.

First, a milk material, water according to the need, other components and the like are mixed, preferably homogenized and heat sterilized. The homogenization and the heat sterilization can be conducted by general methods. A lactic acid bacterium starter is added (seeded) to the sterilized modified milk solution after the heat sterilization, and the solution is kept at a certain fermentation temperature and fermented. A fermented product is thus obtained. Through fermentation, curd is formed.

As the lactic acid bacterium starter, for example, lactic acid bacteria which are generally used for yogurt production, such as *Lactobacillus bulgaricus, Lactococcus lactis* and *Streptococcus thermophilus,* can be used. When the pH reaches the target value, the formed curd is crushed by stirring and cooled to 10°C or lower, and a fermented product is thus obtained. By cooling to 10°C or lower, the activity of the lactic acid bacterium can be reduced, and the formation of an acid can be inhibited.

Next, the fermented product obtained by the fermentation process is heat treated, and a post-heating fermented product (the fermented product after the heat treatment) is thus obtained. By appropriately heating the fermented product, the formation of an acid by the lactic acid bacterium in the post-heating fermented product can be inhibited. As a result, a decrease in pH during the subsequent production process and/or during the storage of the concentrated fermented milk containing a bifidobacterium or a lactic acid bacterium can be inhibited, and as a result, the viability of the bifidobacterium or the lactic acid bacterium can be improved.

Next, *Bifidobacterium breve* M-16V (NITE BP-02622), *Bifidobacterium breve* MCC1274 (FERM BP-11175), *Bifidobacterium longum* subsp. *infantis* M-63 (NITE BP-02623) or *Lactobacillus paracasei* MCC1849 (NITE BP-01633) is added to the post-heating fermented product obtained by the heat treatment process. The amount to be added based on the post-heating fermented product is preferably 1×10⁴ to 1×10¹³ cfu/ml, more preferably 1×10⁵ to 1×10¹² cfu/ml. In the case of dead cells, cfu/ml can be replaced with cells/ml.

Then, concentration is conducted. The concentration process can be conducted appropriately using a known concentration method. For example, a centrifugation method or a membrane separation method can be used.

An effect of inhibiting norovirus infection can be expected through the intake of the fermented milk obtained as described above. Moreover, the fermented milk can be used for preventing or treating a syndrome or a symptom which can be prevented or treated by inhibiting norovirus infection. Examples of the syndrome or the symptom include nausea, vomiting, diarrhea, abdominal pain, fever, and the like.

## Claims

1. A composition for inhibiting norovirus infection comprising one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* as an active ingredient.

2. The composition for inhibiting norovirus infection according to claim 1, wherein *Bifidobacterium breve* is *Bifidobacterium breve* NITE BP-02622 or *Bifidobacterium breve* FERM BP-11175.

3. The composition for inhibiting norovirus infection according to claim 1 or 2, wherein *Bifidobacterium longum* subsp. *infantis* is *Bifidobacterium longum* subsp. *infantis* NITE BP-02623.

4. The composition for inhibiting norovirus infection according to any one of claims 1 to 3, wherein *Lactobacillus paracasei* is *Lactobacillus paracasei* NITE BP-01633.

5. The composition for inhibiting norovirus infection according to any one of claims 1 to 4 which comprises lactoferrin.

6. The composition for inhibiting norovirus infection according to any one of claims 1 to 5 which is a pharmaceutical composition.

7. The composition for inhibiting norovirus infection according to any one of claims 1 to 5 which is a food or drink composition.

8. Use of one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* in the manufacture of a composition for inhibiting norovirus infection.

9. Use of one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for the inhibition of norovirus infection.

10. One or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for use in the inhibition of norovirus infection.

11. One or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* for use in the prevention or treatment of a syndrome or a symptom which can be prevented or treated by inhibiting norovirus infection.

12. A method for inhibiting norovirus from infecting a human, including a step of administering one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* to a human.

13. A method for preventing or a method for treating a syndrome or a symptom which can be prevented or treated by inhibiting norovirus infection, including a step of administering one or more kinds of bacteria selected from the group consisting of *Bifidobacterium breve, Bifidobacterium longum* subsp. *infantis, Bifidobacterium animalis, Bifidobacterium bifidum, Bifidobacterium catenulatum, Lactobacillus gasseri, Lactobacillus helveticus, Lactobacillus delbrueckii* subsp. *bulgaricus* and *Lactobacillus paracasei* to a human.
